# EUROPEAN PATENT SPECIFICATION

(11) **EP 2 987 397 B1**
(45) Date of publication and mention of the grant of the patent: **07.12.2016**
(21) Application number: 13882303.4
(22) Date of filing: 18.04.2013
(51) Int. Cl.: A01G 1/00, A01G 7/00, G06Q 50/02, A01K 29/00, G01N 33/00

(54) **VEGETATION DETERMINATION PROGRAM, VEGETATION DETERMINATION DEVICE AND VEGETATION DETERMINATION METHOD**
VEGETATIONSBESTIMMUNGSPROGRAMM, VEGETATIONSBESTIMMUNGSVORRICHTUNG UND VEGETATIONSBESTIMMUNGSVERFAHREN
PROGRAMME, DISPOSITIF ET PROCÉDÉ DE DÉTERMINATION DE VÉGÉTATION

(43) Date of publication of application: 24.02.2016
(73) Proprietor: Fujitsu Limited, Kawasaki-shi, Kanagawa 211-8588 (JP)
(72) Inventor: MOTOSHIMA, Toshimi, Fukuoka-shi Fukuoka 814-8589 (JP); KANAMORI, Akihito, Fukuoka-shi Fukuoka 814-8589 (JP); OKAMOTO, Kouki, Fukuoka-shi Fukuoka 814-8589 (JP); YAMANO, Daiji, Kawasaki-shi Kanagawa 211-8588 (JP)
(74) Representative: Hoffmann Eitle
(86) International application number: PCT/JP2013/061549
(87) International publication number: WO 2014/170996

(56) References cited:
- JP-A- S6 488 350
- JP-A- H11 128 210

## Description

### TECHNICAL FIELD

The present invention relates to a vegetation assessing program, a vegetation assessing apparatus, and a vegetation assessing method.

### BACKGROUND ART

When cattle are fed, roughage high in fiber such as fresh grass, silage, hay, rice straw, etc. has to be provided in addition to concentrated feed. As one means of providing roughage, cattle are pastured in a field, where the cattle can graze freely on grass, i.e., roughage.

Cattle have grass preferences and if a grass of low preference increases in a pasture, the cattle may fail to consume a sufficient volume of grass, negatively affecting productivity. Thus, cattle raisers manage pastures to ensure sufficient growth of feed grasses such as by sowing seeds of grasses highly preferred and suitable for the pastures. Further, since vegetation varies according to climate and changes in the weather, cattle raisers periodically look around the pastures to observe the vegetation. As a related technology, there is a device that manages the amount of movement of an animal by using step counts.

Patent Document 1: Japanese Laid-Open Patent Publication No. H11-12821

### DISCLOSURE OF INVENTION

### PROBLEM TO BE SOLVED BY THE INVENTION

Nonetheless, with conventional techniques, a problem arises in that a large work burden is placed on cattle raisers who have to take a look at the pasture to observe the vegetation and assess the state of the vegetation of the pasture.

According to one aspect, an object of the present invention is to provide a vegetation assessing program, a vegetation assessing apparatus, and a vegetation assessing method that can assess the state of vegetation of a pasture.

### MEANS FOR SOLVING PROBLEM

According to one aspect of the present invention, a vegetation assessing program, a vegetation assessing apparatus, and a vegetation assessing method are proposed that assess whether a state of vegetation of a pasture is a state suitable for pasturing, based on temporal change of step count measurement results obtained by a step measuring device attached to a livestock animal pastured in the pasture; and output an obtained assessment result.

### EFFECT OF THE INVENTION

According to one aspect of the present invention, an effect is achieved in that the state of vegetation of a pasture can be assessed.

### BRIEF DESCRIPTION OF DRAWINGS

FIG. 1 is a diagram depicting an example of a vegetation assessing method according to an embodiment;
FIG. 2 is a diagram depicting an example of system configuration of a rearing support system 200;
FIG. 3 is a block diagram depicting an example of hardware configuration of a vegetation assessing apparatus 101;
FIG. 4 is a block diagram of an example of hardware configuration of a client apparatus 201;
FIG. 5 is a block diagram of an example of hardware configuration of a communications apparatus 203;
FIG. 6 is a diagram depicting an example of contents of a measurement result table 260;
FIG. 7 is a diagram depicting an example of contents of a step count table 230;
FIG. 8 is a diagram depicting an example of contents of a threshold table 240;
FIG. 9 is a diagram depicting an example of contents of a correction value table 250;
FIG. 10 is a block diagram of an example of a functional configuration of the vegetation assessing apparatus 101;
FIG. 11 is a diagram depicting an example of contents of a step count average table 1100;
FIG. 12 is a diagram depicting a screen example of information depicted on a display 406;
FIG. 13 is a flowchart depicting an example of a procedure of an obtaining process of the vegetation assessing apparatus 101;
FIG. 14 is a flowchart depicting an example of a procedure of a vegetation assessing process of the vegetation assessing apparatus 101; and
FIG. 15 is a flowchart depicting an example of a procedure of an estrus assessing process.

### BEST MODE(S) FOR CARRYING OUT THE INVENTION

Embodiments of a vegetation assessing program, a vegetation assessing apparatus, and a vegetation assessing method according to the present invention will be described in detail with reference to the accompanying drawings.

### (Example of vegetation assessing method)

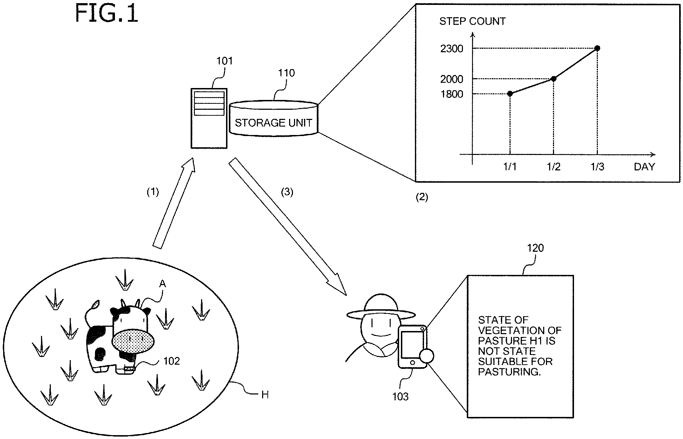

FIG. 1 is a diagram depicting an example of the vegetation assessing method according to an embodiment. In FIG. 1, a vegetation assessing apparatus 101 is a computer that assesses the state of vegetation of a pasture. Here, a pasture is a place where livestock animals are released and kept. Livestock animals are, for example, cattle, pigs, and horses that are raised. Vegetation is plants growing in a pasture.

When livestock animals are fed, in addition to concentrated feed, roughage has to be provided to improve meat quality or milk quality, or to maintain proper motility in the ruminant stomach. Concentrated feed is feed that is low in fiber and water, and high in protein, fat, carbohydrates, etc. such as, for example, rice bran, soy beans, corn, etc. Roughage high in fiber such as, for example, fresh grass, silage, hay, rice straw, etc. is also feed.

Livestock animals have grass preferences and if a grass of a low preference increases in a pasture, the livestock animals may fail to consume a sufficient volume of grass, negatively affecting productivity. Thus, a livestock raiser manages pastures to ensure sufficient growth of highly preferred grasses by sowing seeds of grasses that are highly preferred by the livestock, while assessing the pasture vegetation, which varies with the climate and changes in the weather.

Nonetheless, the greater the number of livestock animals is, the larger the pasture has to be. Further, pastures managed by a livestock raiser may be located at multiple locations. Therefore, it is difficult for the livestock raiser to periodically take a look around the pastures to observe and assess the pasture vegetation, which changes with the climate and changes in the weather.

In this regard, in the present embodiment, the vegetation assessing apparatus 101 assesses the state of the pasture vegetation from upward trends in step counts measured by step measuring devices attached to livestock animals pastured in a pasture and outputs the state, thereby enabling the livestock raiser to determine the state of the vegetation without having to look around the pasture. Hereinafter, an example of a vegetation assessing process of the vegetation assessing apparatus 101 will be described.
(1) The vegetation assessing apparatus 101 obtains a step count measurement result of a step measuring device 102 that is attached to a livestock animal A pastured in a pasture H. More specifically, for example, the vegetation assessing apparatus 101 obtains step count measurement results obtained for a given time interval by the step measuring device 102.
   Here, the given time interval is, for example, in units of hours, days, weeks, etc. Obtained step count measurement results, for example, are stored to a storage unit 110 of the vegetation assessing apparatus 101. In the example depicted in FIG. 1, the daily step count measurement result obtained by the step measuring device 102 from January 1 to January 3 are stored in the storage unit 110.
(2) The vegetation assessing apparatus 101 assesses whether the state of the vegetation of the pasture H is a suitable state, based on temporal changes of the step count measurement results obtained by the step measuring device 102. Here, in a pasture in which an abundance of highly preferred grass is growing, the livestock can consume a sufficient volume of grass and therefore, the rumination time of the livestock tends to increase. Rumination is an action of returning swallowed food to the mouth, re-chewing and re-swallowing the food.
   On the other hand, if there is little highly preferred grass or highly preferred grass becomes depleted, the rumination time becomes shorter and since the livestock searches for preferred grass, the step count of the livestock tends to increase. In other words, when the step counts measured at the given time interval tends to increase with the passage of time, it can be determined that the state of the pasture vegetation tends to be declining.
   Thus, the vegetation assessing apparatus 101, for example, refers to the storage unit 110 and if the daily step count increases with the passage of time, assesses that the state of the vegetation of the pasture H is not a suitable state. In the example depicted in FIG. 1, with the passage of each day, the daily step count is increasing. In this case, the vegetation assessing apparatus 101 assesses that the state of the vegetation of the pasture is not a suitable state.
(3) The vegetation assessing apparatus 101 outputs the assessment result. More specifically, for example, the vegetation assessing apparatus 101 transmits the assessment result to a computer used by the livestock raiser. In the example depicted in FIG. 1, as a result of transmitting the assessment result to a portable communications terminal 103 used by the livestock raiser, an alarm screen 120 indicating that the state of the vegetation of the pasture H is not a suitable state is displayed at the communications terminal 103.

In this manner, the vegetation assessing apparatus 101 enables the state of the vegetation of the pasture H to be assessed based on temporal changes in the step count measurement results obtained by the step measuring device 102 attached to the livestock animal A. Thus, the livestock raiser can determine the state of the vegetation of the pasture H without having to go to the pasture H to observe the vegetation, enabling the work load of the livestock raiser to be reduced.

### (Example of system configuration of rearing support system 200)

An example of system configuration of a rearing support system 200 according to an embodiment will be described. In the description hereinafter, description will be given taking cattle such as beef cattle and dairy cows as an example of livestock animals. Further, grass of high preference to the livestock animals may be indicated as "feed grass" and grass of low preference to the livestock animals may be indicated as "weeds".

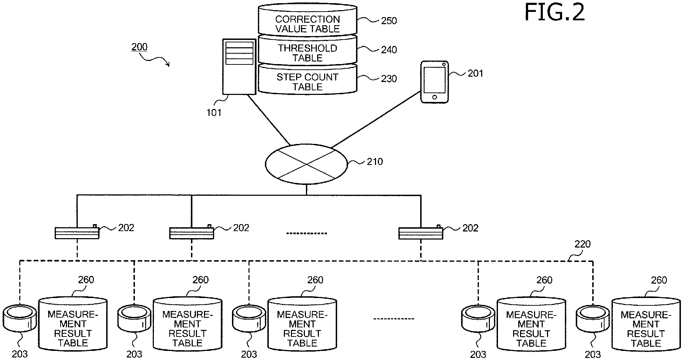

FIG. 2 is a diagram depicting an example of system configuration of the rearing support system 200. In FIG. 2, the rearing support system 200 includes the vegetation assessing apparatus 101, a client apparatus 201, and a relay apparatus 202. In the rearing support system 200, the vegetation assessing apparatus 101, the client apparatus 201, and the relay apparatus 202 are connected via a cabled or wireless network 210. The network 210, for example, is a local area network (LAN), a wide area network (WAN), the Internet, etc.

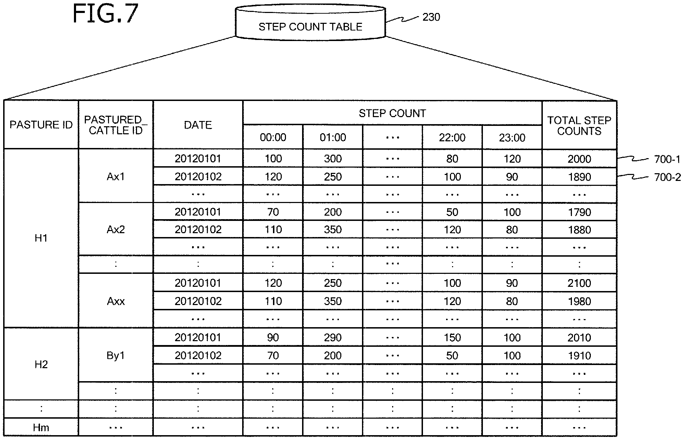

Here, the vegetation assessing apparatus 101 has a step count table 230, a threshold table 240, and a correction value table 250, and assesses the state of the vegetation of pastures H1 to Hm (m: natural number of 1 or greater). The pastures H1 to Hm are pastures managed by a livestock raiser. More specifically, for example, the vegetation assessing apparatus 101 is a server included in a cloud computing system. Contents of the various tables 230, 240, and 250 will be described hereinafter with reference to FIGs. 7 to 9.

In the description hereinafter, an arbitrary pasture among the pastures H1 to Hm may be indicated as "pasture Hj" (j= 1, 2, ..., m). Further, plural cattle pastured in a pasture Hj may indicated as "cattle A1 to An" (n: natural number of 1 or greater), and an arbitrary cattle among the cattle A1 to An may be indicated as "cattle Ai" (i= 1, 2, ..., n).

The client apparatus 201 is a computer used by the livestock raiser. More specifically, for example, the client apparatus 201 is a smartphone, a mobile telephone, a tablet personal computer (PC), a Personal Handy-phone System (PHS) device, a PC, a note PC, etc. The communications terminal 103 depicted in FIG. 1, for example, corresponds to the client apparatus 201.

The relay apparatus 202 is a computer that has a communications function. The relay apparatus 202, for example, is installed in each pasture Hj. The relay apparatus 202 is connected to a communications apparatus 203 via a wireless network 220, and relays communication of the communications apparatus 203 and the vegetation assessing apparatus 101.

The communications apparatus 203 is a computer that has a measurement result table 260 and wirelessly communicates with the relay apparatus 202 via the wireless network 220. Further, the communications apparatus 203 is attached to, for example, an ankle of a cattle Ai and has a function of measuring a step count of the cattle Ai. The step measuring device 102 depicted in FIG. 1, for example, corresponds to the communications apparatus 203.

The communications apparatus 203 further transmits obtained step count measurement results to the vegetation assessing apparatus 101, via the relay apparatus 202. More specifically, for example, the communications apparatus 203 refers to the measurement result table 260, and transmits to the vegetation assessing apparatus 101, the step count measurement result obtained for a given time interval t. The given time interval t, for example, is 1 hour, 1 day, etc.

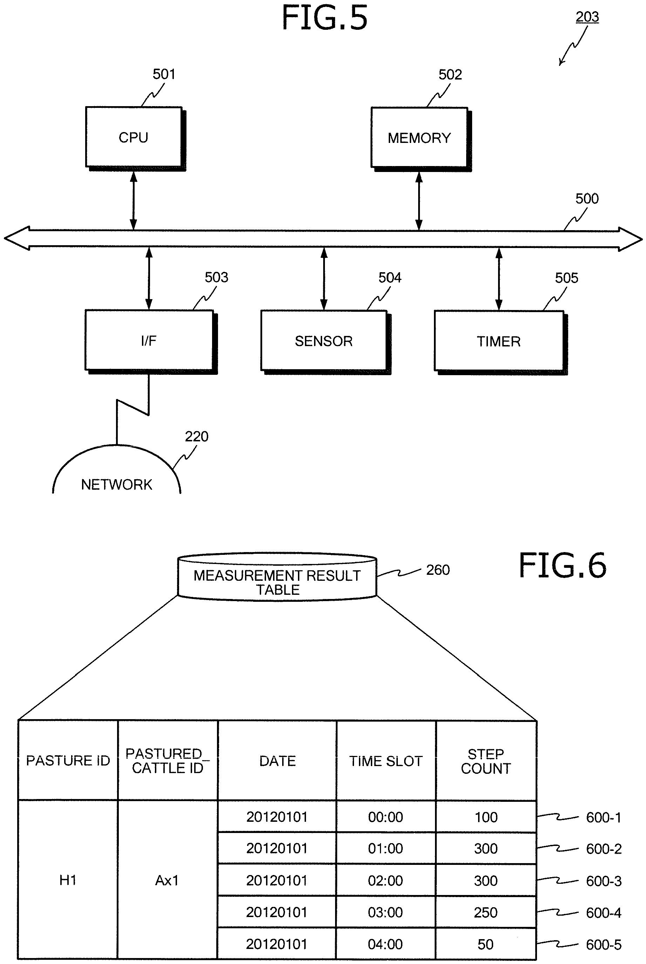

In the description hereinafter, the given time interval t is assumed to be "1 hour". Further, configuration may be such that in response to a transmission request from the vegetation assessing apparatus 101, the communications apparatus 203 transmits a step count measurement result to the vegetation assessing apparatus 101. Contents of the measurement result table 260 will be described with reference to FIG. 6.

Further, the communications apparatus 203, for example, may be attached to each member of a herd in the pasture Hj, or a member selected from the herd (e.g., the alpha). However, compared to adult cattle, calves tend to scamper about and have a high step count and consequently, may be excluded from being subject to attachment of the communications apparatus 203.

### (Example of hardware configuration of vegetation assessing apparatus 101)

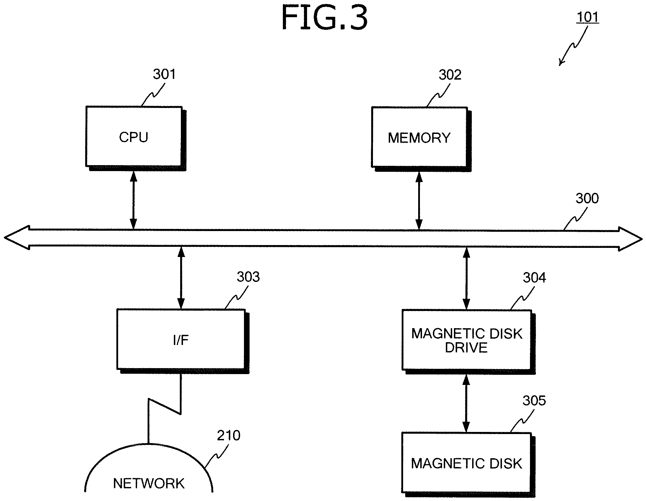

FIG. 3 is a block diagram depicting an example of hardware configuration of the vegetation assessing apparatus 101. In FIG. 3, the vegetation assessing apparatus 101 has a central processing unit (CPU 301), memory 302, an interface (I/F) 303, a magnetic disk drive 304, and a magnetic disk 305, respectively connected by a bus 300.

Here, the CPU 301 governs overall control of the vegetation assessing apparatus 101. The memory 302, for example, includes read-only memory (ROM), random access memory (RAM), and flash ROM. More specifically, for example, the flash ROM and ROM store various types of programs; and the RAM is used as a work area of the CPU 301. The programs stored in the memory 302 are loaded onto the CPU 301, whereby coded processes are executed by the CPU 301.

The I/F 303 is connected to the network 210 through a communications line, and to other computers (e.g., the client apparatus 201 and the relay apparatus 202 depicted in FIG. 2) via the network 210. The I/F 303 administers an internal interface with the network 210 and controls the input and output of data from other computers. A modem or LAN adapter, for example, can be used as the I/F 303.

The magnetic disk drive 304, under the control of the CPU 301, controls the reading and writing of data with respect to the magnetic disk 305. The magnetic disk 305 stores data written thereto under the control of the magnetic disk drive 304.

The vegetation assessing apparatus 101 may have a solid state drive (SSD), a keyboard, a mouse, a display, etc. in addition to the above components. Further, the relay apparatus 202 depicted in FIG. 2 can also be realized by, for example, by a hardware configuration similar to that of the vegetation assessing apparatus 101 described above.

### (Example of hardware configuration of client apparatus 201)

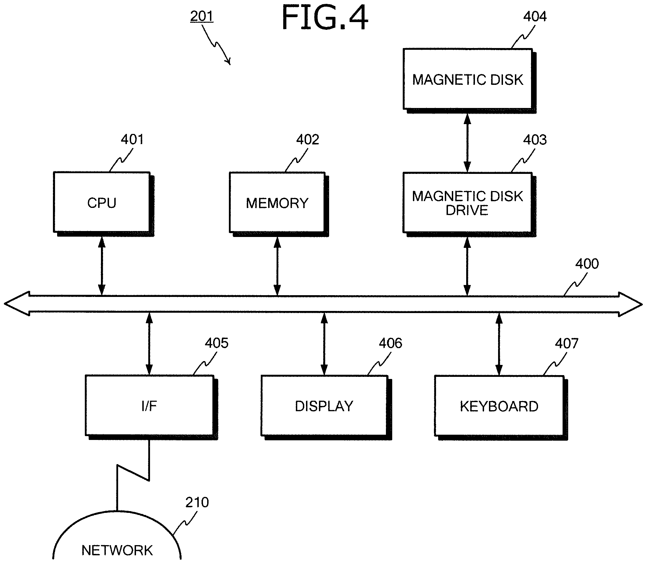

FIG. 4 is a block diagram of an example of hardware configuration of the client apparatus 201. In FIG. 4, the client apparatus 201 has a CPU 401, memory 402, a magnetic disk drive 403, a magnetic disk 404, an I/F 405, a display 406, and a keyboard 407, respectively connected by a bus 400.

Here, the CPU 401 governs overall control of the client apparatus 201. The memory 402, for example, includes ROM, RAM, etc. More specifically, for example, the ROM stores various types of programs and the RAM is used as a work area of the CPU 401. The magnetic disk drive 403, under the control of the CPU 401, controls the reading and writing of data with respect to the magnetic disk 404. The magnetic disk 404 stores the data written thereto under the control of the magnetic disk drive 403.

The I/F 405 is connected to the network 210 through a communications line, and is connected to other computers (e.g., the vegetation assessing apparatus 101) via the network 210. The I/F 405 administers an internal interface with the network 210 and controls the input and output of data from other computers.

The display 406 displays text, images, functional information, etc. in addition to a cursor, icons, and toolboxes. For example, a liquid crystal display, an organic electroluminescence (EL) display, etc. can be used as the display 406. The keyboard 407 has keys for inputting text, numerals, various instructions, etc. and performs data input. The keyboard 407, for example, may be a touch panel input pad, a numeric pad, etc.

### (Example of hardware configuration of communications apparatus 203)

FIG. 5 is a block diagram of an example of hardware configuration of the communications apparatus 203. In FIG. 5, the communications apparatus 203 has a CPU 501, memory 502, an I/F 503, a sensor 504, and a timer 505, respectively connected by a bus 500.

Here, the CPU 501 governs overall control of the communications apparatus 203. The memory 502 includes ROM, RAM, etc. More specifically, for example, the ROM stores various types of programs and the RAM is used as a work area of the CPU 501.

The I/F 503 is connected to the wireless network 220 and is connected to other computers (e.g., the relay apparatus 202) via the wireless network 220. The I/F 503 administers an internal interface with the wireless network 220 and controls the input and output of data from other computers.

The sensor 504 outputs information for detecting a behavior of the communications apparatus 203. For example, when the sensor 504 is implemented by a gyro sensor, a triple axis accelerometer, etc. and the communications apparatus 203 accelerates, the sensor 504 outputs information corresponding to the acceleration that occurred. The timer 505 has a function of measuring time. More specifically, for example, the timer 505 measures the actual time. The timer 505 may further measure the time that elapses from a given time point.

In addition to the components described above, the communications apparatus 203 may further include, for example, a global positioning system (GPS) unit that receives radio signals from satellites and outputs GPS information indicating the current position of the apparatus thereof.

### (Contents of measurement result table 260)

Contents of the measurement result table 260 depicted in FIG. 2 will be described. The measurement result table 260, for example, is realized by the memory 502 of the communications apparatus 203 depicted in FIG. 5. Here, description will be given taking the measurement result table 260 of the communications apparatus 203 attached to a given cattle in the pasture H1 as an example.

FIG. 6 is a diagram depicting an example of the contents of the measurement result table 260. In FIG. 6, the measurement result table 260 has fields for pasture IDs, pastured_cattle IDs, dates, time slots, and step counts. By setting information into each of the fields, the 5 most recent measurement data (e.g., measurement data 600-1 to 600-5) are stored as records.

Here, a pasture ID is an identifier that identifies the pasture Hj in which a cattle Ai to which the communications apparatus 203 is attached is pastured. A pastured_cattle ID is an identifier that identifies the cattle Ai to which the communications apparatus 203 is attached. Dates indicate the year, month, day when each step count was measured for the cattle Ai to which the communications apparatus 203 is attached.

Time slots are time slots during which the step counts were measured for the cattle Ai to which the communications apparatus 203 is attached. For example, 00:00 represents a time slot from 00:00.00 to 00:59.59. Each step count is a step count measurement result obtained for the cattle Ai by the communications apparatus 203. For example, measurement data 600-1 indicates a step count of "100" for the 00:00 time slot on 2012/1/1, for a cattle Ax1 pastured in the pasture H1.

Here, an example of process details of the communications apparatus 203 that measures the step count of a cattle Ai will be described. The communications apparatus 203 initializes a measured value C as "C=0" when the actual time measured by the timer 505 (refer to FIG. 5) becomes a reference time point. The reference time point, for example, is set to 00 minutes 00 seconds of each time slot. The actual time measured by the timer 505 of each of the communications apparatuses 203 is assumed to be synchronized.

The communications apparatus 203 increments the measured value C when the sensor 504 (refer to FIG. 5) detects acceleration that occurs with each step, at the instant that a cattle Ai takes a step. When the actual time measured by the timer 505 becomes the reference time point, the communications apparatus 203 sets the date, the time slot, and the measured value C into the respective fields for the date, the time slot, and the step count in the measurement result table 260, and initializes the measured value C as "x=0".

As a result, new measurement data are stored as a record in the measurement result table 260. In this case, the communications apparatus 203 deletes the oldest measurement data stored in the measurement result table 260. Thus, the communications apparatus 203 can measure a step count of a cattle Ai hourly.

The communications apparatus 203 further transmits measurement results stored in the measurement result table 260 to the vegetation assessing apparatus 101, via the relay apparatus 202, for example. In this case, the communications apparatus 203 may transmit the 5 most recent measurement data (e.g., measurement data 600-1 to 600-5) stored to the measurement result table 260, or may transmit the single most recent measurement data (e.g., measurement data 600-5).

Transmission timing of the measurement data can be set arbitrarily. More specifically, for example, configuration may be such that when new measurement data is stored to the measurement result table 260, the communications apparatus 203 transmits the measurement data stored in the measurement result table 260 to the vegetation assessing apparatus 101. Thus, step count measurement data of a cattle Ai can be transmitted to the vegetation assessing apparatus 101 hourly.

Although description has been given where the 5 most recent measurement data are stored in the measurement result table 260, the number of the most recent measurement data to be stored in the measurement result table 260 can be set arbitrarily.

### (Contents of step count table 230)

Contents of the step count table 230 depicted in FIG. 2 will be described. The step count table 230, for example, is realized by the memory 302 of the vegetation assessing apparatus 101 depicted in FIG. 3.

FIG. 7 is a diagram depicting an example of the contents of the step count table 230. In FIG. 7, the step count table 230 has fields for pasture IDs, pastured_cattle IDs, dates, step counts, and total step counts. By setting information into each of the fields, step count data (e.g., step count data 700-1, 700-2) are stored as records.

Here, a pasture ID is an identifier that identifies a pasture Hj. A pastured_cattle ID is an identifier that identifies a cattle Ai pastured in a pasture Hj. Each date indicates the year, month, day when a step count was measured for a cattle Ai. Each step count is a step count measured for a cattle Ai, for each time slot 00:00 to 23:00. Each total step count is a total step count for 1 day, for a cattle Ai. A total step count can be obtained by cumulating the step counts of each time slot 00:00 to 23:00.

For example, a step count for each time slot 00:00 to 23:00 and a total step count of "2000" on 2012/1/1 can be specified for the cattle Ax1 pastured in the pasture H1, by step count data 700-1. The number of days of step count data to be stored in the step count table 230 can be arbitrarily set.

### (Contents of threshold table 240)

Contents of the threshold table 240 depicted in FIG. 2 will be described. The threshold table 240, for example, is stored in the memory 302 of the vegetation assessing apparatus 101.

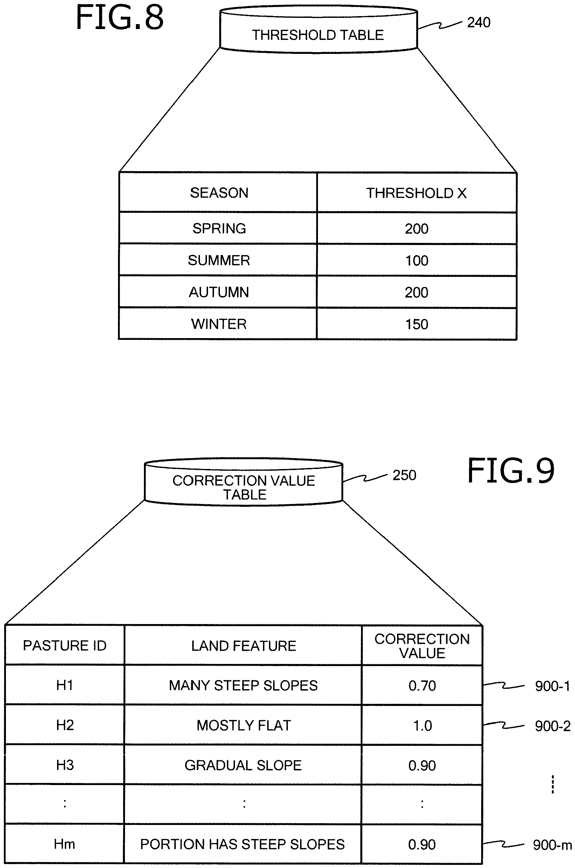

FIG. 8 is a diagram depicting an example of the contents of the threshold table 240. In FIG. 8, the threshold table 240 stores a threshold X for each season. In this example, an interval from March to May 3 is assumed to be "spring"; an interval from June to August is assumed to be "summer"; an interval from September to November is assumed to be "autumn"; and an interval from December to February is assumed to be "winter".

Here, the threshold X is a value used to assess the state of the vegetation of a pasture Hj. In this example, the threshold X for summer when plants that have grown large make walking difficult and the threshold X for winter when walking is difficult when snow accumulates are set to low values compared to the thresholds X for spring and autumn. Nonetheless, a common value may be used for the threshold X throughout the year.

### (Contents of correction value table 250)

Contents of the correction value table 250 depicted in FIG. 2 will be described. The correction value table 250, for example, is stored in the memory 302 of the vegetation assessing apparatus 101.

FIG. 9 is a diagram depicting an example of the contents of the correction value table 250. In FIG. 9, the correction value table 250 has fields for pasture IDs, land features, and correction values. By setting information into each of the fields, correction value data 900-1 to 900-m are stored as records.

Here, a pasture ID is an identifier that identifies a pasture Hj. A land feature is a land feature that characterizes a pasture Hj. A correction value is a value for correcting the step count of a cattle Ai pastured in a pasture Hj, according to the land features of the pasture Hj. For example, correction value data 900-1 indicates land features "many steep slopes" and a correction value "0.70" for the pasture H1.

### (Example of functional configuration of vegetation assessing apparatus 101)

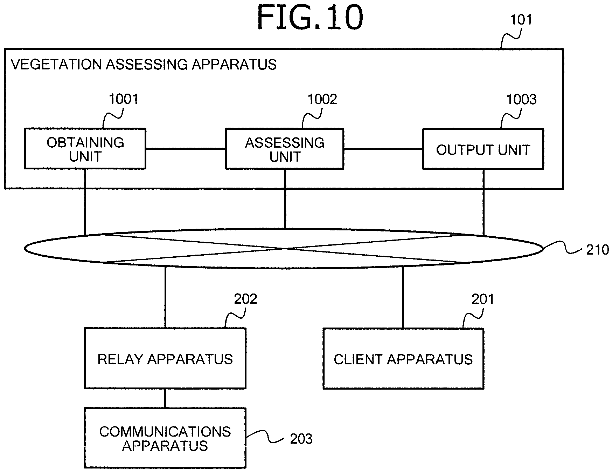

FIG. 10 is a block diagram of an example of a functional configuration of the vegetation assessing apparatus 101. In FIG. 10, the vegetation assessing apparatus 101 is configured to include an obtaining unit 1001, an assessing unit 1002, and an output unit 1003. The obtaining unit 1001 to the output unit 1003 are functions forming a control unit and more specifically, for example, these functions are realized by executing on the CPU 301, a program stored in a storage apparatus such as the memory 302, the magnetic disk 305, etc. depicted in FIG. 3, or by the I/F 303. Further, process results of the respective functional units, for example, are stored to a storage apparatus such as the memory 302, the magnetic disk 305, etc.

The obtaining unit 1001 has a function of obtaining measurement data that includes step count measurement results obtained by the communications apparatus 203 attached to a cattle Ai pastured in a pasture Hj. Here, a step count measurement result, for example, is the measured value C of the step count measured during the given time interval t. Further, measurement data may include, for example, information indicating the date and time of measurement of the step count, the pastured_cattle ID of the cattle Ai, information related to the location of the cattle Ai, in addition to the step count measurement result.

The information related to the location of the cattle Ai, for example, is the pasture ID of the pasture Hj in which the cattle Ai pastured. When the communications apparatus 203 has a GPS unit, GPS information indicating the current position of the communications apparatus 203 may be included in the measurement data as information related to the location of the cattle Ai. The measurement data, for example, is the measurement data 600-1 to 600-5 depicted in FIG. 6.

More specifically, for example, via the network 210, the obtaining unit 1001 receives from the relay apparatus 202, measurement data that includes step count measurement results obtained by the communications apparatus 203. Further, for example, the obtaining unit 1001 may obtain the measurement data, which includes step count measurement results obtained by the communications apparatus 203, consequent to an input operation by the user using a non-depicted keyboard or mouse.

The obtaining unit 1001 may further classify the step count measurement results based on information that is included in the obtained measurement data and related to the location of the cattle Ai. More specifically, for example, the obtaining unit 1001 classifies the step count measurement results for each pasture Hj indicated by the information related to the location of the cattle Ai. The obtained measurement data, for example, is stored to the step count table 230 depicted in FIG. 7.

The assessing unit 1002 has a function of assessing based on temporal changes of the step measurement results included in the measurement data obtained by the obtaining unit 1001, whether the state of the vegetation of the pasture Hj is a suitable state for pasturing. More specifically, for example, when the step counts for each given time interval T increase with the elapse of time, the assessing unit 1002 assesses that the state of the vegetation of the pasture Hj is not suitable for pasturing.

The given time interval T, for example, is 1 hour, 1 day, 1 week, 1 month, etc. However, pastured cattle tend to exhibit a behavior of ruminating when ingesting feed grass and sleeping after ruminating, multiple times during a single day. The rumination time, for example, is 1 hour. Further, the sleeping period, for example, is 2 hours.

In other words, the several hours from when a pastured cattle begins rumination until the pastured cattle wakes up occupy time slots during which the step count of the pastured cattle does not increase for the most part. Further, the time slots when a pastured cattle performs the series of behaviors including ruminating and sleeping during a single day vary day-by-day. Therefore, the time interval T, for example, is preferably set as a time interval on the order of 1 day or 1 week.

More specifically, for example, the assessing unit 1002 may assess that the state of the vegetation of a pasture Hj is not suitable for pasturing, when the value of the step count measurement results of a first interval less the step count measurement results of a second interval that is before the first interval is a given threshold or greater. Here, the first and the second intervals are intervals separated by the given time interval T.

Hereinafter, description will be given using an example in which the time interval T is assumed to be "1 day (24 hours)". Further, a day when assessment of whether the state of the vegetation of a pasture Hj is a suitable state for pasturing is performed may be indicated as "assessment day".

In this case, the assessing unit 1002, for example, may assess that the state of the vegetation of a pasture Hj is not a state suitable for pasturing, when a difference d of a step count S for the assessment day less a reference step count S_{b} is the threshold X or greater. On the other hand, the assessing unit 1002 may assess that the state of the vegetation of pasture Hj is a state suitable for pasturing, when the difference d is less than the threshold X.

Here, the step count S, for example, may be an average of the total step counts of 1 day for the cattle A1 to An pastured in a pasture Hj, or an average of the total step counts of 1 day for plural heads of cattle selected from the cattle A1 to An. Further, for example, the step count S further may be the total step count of 1 day for a specific cattle among the cattle A1 to An. The specific cattle, for example, may be the highest ranking alpha in the herd, or a lowest ranking cattle in the herd.

The reference step count S_{b} is a value that is based on the step count S of another day that is before the assessment day. The reference step count S_{b}, for example, may be the step count S of the day before the assessment day, or the step count S of the day when pasturing in a pasture Hj begins. The reference step count S_{b} may be an average of the step counts S of several days after pasturing in the pasture Hj begins, among the days before the assessment day.

Immediately after release of the cattle Ai into the pasture Hj, the step count thereof tends to increase consequent to surveillance of the pasture Hj. However, 2 or 3 days after pasturing begins, variation of the step count of the cattle Ai settles down. Therefore, the reference step count S_{b} may be the step count S a few days after pasturing in the pasture Hj begins, among the days before the assessment day.

The threshold X, for example, is set to a value that enables determination that when the step count S of the assessment day, with respect to the step count S_{b}, increases to the threshold X or more, there is little feed grass in the pasture Hj, or since there is no feed grass, the pastured cattle are searching for preferred grass. The threshold X, for example, can be specified from the threshold table 240 depicted in FIG. 8.

For example, when the assessment day is during an interval from March to May, the assessing unit 1002 refers to the threshold table 240 and specifies the threshold X "X=200" of the season "spring". Thus, if there is little feed grass in the pasture Hj or if the feed grass has been depleted, and the step count of the pastured cattle rapidly increases, it can be assessed that the state of the vegetation of the pasture Hj is not a state suitable for pasturing.

The assessing unit 1002 may assess whether the state of the vegetation of the pasture Hj is a state suitable for pasturing, based on temporal changes of the step count measurement results for the remaining cattle among the cattle A1 to An pastured in the pasture Hj exclusive of cattle of a specified state. Here, cattle of a specified state are cattle in estrus, sick cattle, etc.

Cattle of a specified state have per unit time step counts that tend to increase and decrease compare to cattle of a normal state. For example, cattle in estrus have per unit time step counts that tend to increase compared to cattle of a normal state. Further, sick cattle have per unit step counts that tend to decrease compared to cattle of a normal state. Therefore, the assessing unit 1002 may exclude as processing subjects, the step count measurement results of cattle of a specified state.

Cattle of a specified state, for example, may be specified by operational input by the user using a non-depicted keyboard or mouse. Further, the assessing unit 1002 may refer to the step count table 230 to assess whether a cattle Ai is in a specified state. More specifically, for example, the assessing unit 1002 assesses whether the step count of the cattle Ai satisfies a given condition and if the given condition is satisfied, assesses that the cattle Ai is in a specified state.

The given condition can be set arbitrarily according states such as estrus, illness, etc. For example, when a cattle Ai is assessed to be in estrus, the step count of the cow in estrus increases and using this characteristic, the given condition is set as a condition "step count of cattle Ai is threshold α or greater". The step count of the cattle Ai here, for example, is an average of the per unit time step counts of the cattle Ai, for 1 day. Further, for example, when the cattle Ai is assessed to be sick, the step count of the sick cattle decreases and using this characteristic, the given condition is set as a condition "step count of cattle Ai is less than threshold β".

Further, when the difference d of the step count S of the assessment day less the reference step count S_{b} is the threshold X or greater, the assessing unit 1002 may detect based on the step count measurement results for each time slot of the assessment day, a time slot TZ for which the step count continues to be a given number Q or less for a given period P or longer. Here, the pastured cattle, as described above, tend to perform a series of behaviors including ruminating and sleeping multiple times during a single day.

Further, the number of times that the pastured cattle perform the series of behaviors of ruminating and sleeping is presumed to decrease when there is little feed grass in the pasture Hj. In other words, when the number of times that the series of behaviors of ruminating and sleeping is few, it can be determined that the state of the vegetation of the pasture Hj is not a state suitable for pasturing.

Thus, configuration may be such that when a count k of the detected time slots TZ is less than a given number K, the assessing unit 1002 assesses that the state of the vegetation of the pasture Hj is not a state suitable for pasturing. Further, the assessing unit 1002 may assess that the state of the vegetation of the pasture Hj is suitable for pasturing, when the count k of the detected time slots TZ is the given number K or greater.

The given period P is set to a period that is a sum of the average rumination time and the average sleep time, e.g., 3 hours. Further, the step count of pastured cattle that are ruminating or sleeping is a value nearly 0. Therefore, the given number Q, for example, is set to a value on the order of 10 to 30. Further, the given number K is set to an average count of the number times that pastured cattle perform the series of behaviors of ruminating and sleeping in a single day, e.g., 3 times.

Thus, compared to a case where only the extent of increase of the step counts of each given time interval T is considered, the accuracy in assessing the state of the vegetation of a pasture Hj can be improved. More specifically, for example, even when the step count of a cattle Ai rapidly increases consequent to an external factor such as thunder, the appearance of a natural predator, etc., if the count k of the time slots TZ is the given number K or greater, the state of the vegetation of the pasture Hj can be assessed to be a suitable state for pasturing.

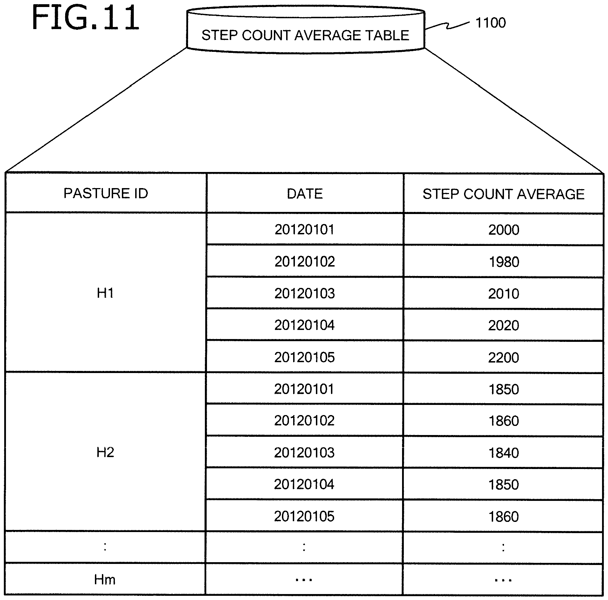

Further, the assessing unit 1002 may assess whether the state of the vegetation of each pasture Hj is a state suitable for pasturing, based on temporal changes in the step count measurement results classified according pasture Hj by the obtaining unit 1001. Thus, the state of vegetation of the respective pastures Hj located at multiple locations can be assessed. Processing details of the assessing unit 1002 will be described hereinafter with reference to FIG. 11.

The output unit 1003 has a function of outputting assessment results obtained by the assessing unit 1002. Forms of output by the output unit 1003, for example, include storage to a storage apparatus such as the memory 302, the magnetic disk 305, etc.; display on a non-depicted display; non-depicted printout; transmission by the I/F 303 to an external computer; etc.

More specifically, for example, the output unit 1003 may generate information indicating that the state of the vegetation of a pasture Hj is a state suitable for pasturing and transmit the information to the client apparatus 201, when the state of the vegetation of the pasture Hj is assessed to be a state suitable for pasturing. As a result, information indicating that the state of the vegetation of the pasture Hj is a state suitable for pasturing is displayed on the display 406 at the client apparatus 201.

Further, the output unit 1003 may generate information indicating that the state of the vegetation of a pasture Hj is not a state suitable for pasturing and transmit the information to the client apparatus 201, when the state of the vegetation at the pasture Hj is assessed to not be a state suitable for pasturing. As a result, information indicating that the state of the vegetation of the pasture Hj is not a state suitable for pasturing is displayed on the display 406 at the client apparatus 201.

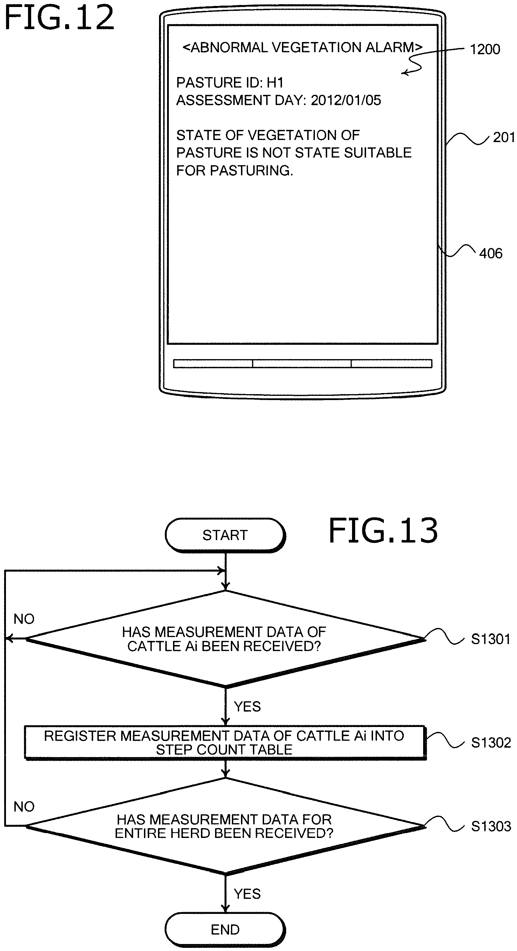

A screen example of information displayed on the display 406 will be described hereinafter with reference to FIG. 12. Further, the address of the client apparatus 201, which is the transmission destination, for example, is correlated with the pasture ID of the pasture Hj managed by the livestock raiser using the client apparatus 201 and stored to a storage apparatus such as the memory 302, the magnetic disk 305, etc.

The reference step count S_{b} may be preset as a common value for the pastures H1 to Hm. More specifically, for example, an averaged total step count of a cattle Ai, for 1 day in the past and measured when the state of the vegetation of the pasture Hj is a state suitable for pasturing may be set as the reference step count S_{b} common to the pastures H1 to Hm.

Nonetheless, bias in the increasing tendency of the step count of the cattle Ai occurs consequent to land features of each pasture Hj and therefore, configuration may be such that step count measurement results are corrected. More specifically, for example, configuration may be such that the assessing unit 1002 refers to the correction value table 250 depicted in FIG. 9 and multiplies the step count measurement results of the cattle Ai by a correction value specific to each pasture Hj to correct the step count measurement results of the cattle Ai. Thus, variability of the increasing tendency of the step counts of the cattle Ai consequent to the land features of each pasture Hj can be reduced.

### (Process details of assessing unit 1002)

An example of process details of the assessing unit 1002 will be described with reference to FIG. 11. Here, description will be given taking as an example, a case where the step count S is assumed to be an average (hereinafter, "step count average") of the total step counts of 1 day, for the cattle A1 to An pastured in a pasture Hj.

The assessing unit 1002 refers to the step count table 230 and for each pasture Hj, calculates a daily step count average of the cattle A1 to An pastured in the pasture Hj. The calculated daily step count average, for example, is stored to a step count average table 1100 depicted in FIG. 11.

FIG. 11 is a diagram depicting an example of the contents of the step count average table 1100. In FIG. 11, the step count average table 1100 has fields for pasture IDs, dates, and step count averages. By setting information into each of the fields, daily step count averages for each pasture Hj are stored.

Here, description will be given taking an example where the assessment day is assumed to be "2012/1/5", the reference step count S_{b} is assumed to be "step count average of day before assessment day", and the state of the vegetation of the pastures H1 and H2 is assessed. In this case, for the pasture H1, the assessing unit 1002 calculates the difference d of the step count average "2200" of the assessment day less the reference step count S_{b} "2020". In this example, the difference d is "d=180".

Further, the assessing unit 1002 refers to the threshold table 240 and identifies the threshold X for the season corresponding to the assessment day. In this example, the season corresponding to the assessment day is winter and therefore, the threshold X is "X=150". The assessing unit 1002 assesses if the difference d is the threshold X or greater. In this example, the difference d is assessed to be the threshold X or greater. In this case, the assessing unit 1002 assesses that the state of the vegetation of the pasture H1 is not a state suitable for pasturing.

Further, for the pasture H2, the assessing unit 1002 calculates the difference d of the step count average "1860" of the assessment day less the reference step count S_{b} "1850". The assessing unit 1002 assesses if the difference d is the threshold X or greater. In this example, the threshold X is "X=150" and therefore, the difference d is assessed to be less than the threshold X. In this case, the assessing unit 1002 assesses that the state of the vegetation of the pasture H2 is a state suitable for pasturing.

### (Screen example of information displayed on display 406)

A screen example of information displayed on the display 406 of the client apparatus 201 will be described. Here, a screen example of information indicating that the state of the vegetation of a pasture Hj is not a state suitable for pasturing will be described.

FIG. 12 is a diagram depicting a screen example of information depicted on the display 406. In FIG. 12, an alarm screen 1200 indicating that the state of the vegetation of the pasture H1 is not a state suitable for pasturing is displayed on the display 406 of the client apparatus 201. Through the alarm screen 1200, the livestock raiser can determine that at the time point of 2012/1/5, the state of the vegetation of the pasture H1 is not a state suitable for pasturing.

### (Procedures of various processes of vegetation assessing apparatus 101)

Procedures of various processes of the vegetation assessing apparatus 101 will be described.

### <Procedure of obtaining process>

A procedure of an obtaining process of the vegetation assessing apparatus 101 will be described. The obtaining process is a process of obtaining measurement data of a cattle Ai pastured in a pasture Hj. The obtaining process, for example, is executed periodically (e.g., 1 hour) for each pasture Hj.

FIG. 13 is a flowchart depicting an example of the procedure of the obtaining process of the vegetation assessing apparatus 101. In the flowchart depicted in FIG. 13, the vegetation assessing apparatus 101 determines whether measurement data of a cattle Ai pastured in a pasture Hj has been received from the relay apparatus 202, via the network 210 (step S1301).

Here, the vegetation assessing apparatus 101 waits for measurement data of a cattle Ai to be received (step S1301: NO). When measurement data of a cattle Ai has been received (step S1301: YES), the vegetation assessing apparatus 101 registers the received measurement data into the step count table 230 (step S1302).

The vegetation assessing apparatus 101 determines whether measurement data for the entire herd of the pasture Hj has been received (step S1303). Here, if measurement data for the entire herd has not been received (step S1303: NO), the vegetation assessing apparatus 101 returns to step S1301. On the other hand, if measurement data for the entire herd has been received (step S1303: YES), the vegetation assessing apparatus 101 ends the series of operations according to the flowchart. Thus, step count measurement results of the cattle A1 to An pastured in a pasture Hj can be obtained.

### <Procedure of vegetation assessing process>

A procedure of the vegetation assessing process of the vegetation assessing apparatus 101 will be described. The vegetation assessing process is a process of assessing whether the state of the vegetation of a pasture Hj is a state suitable for pasturing. The vegetation assessing process, for example, is executed at an arbitrary timing or periodically.

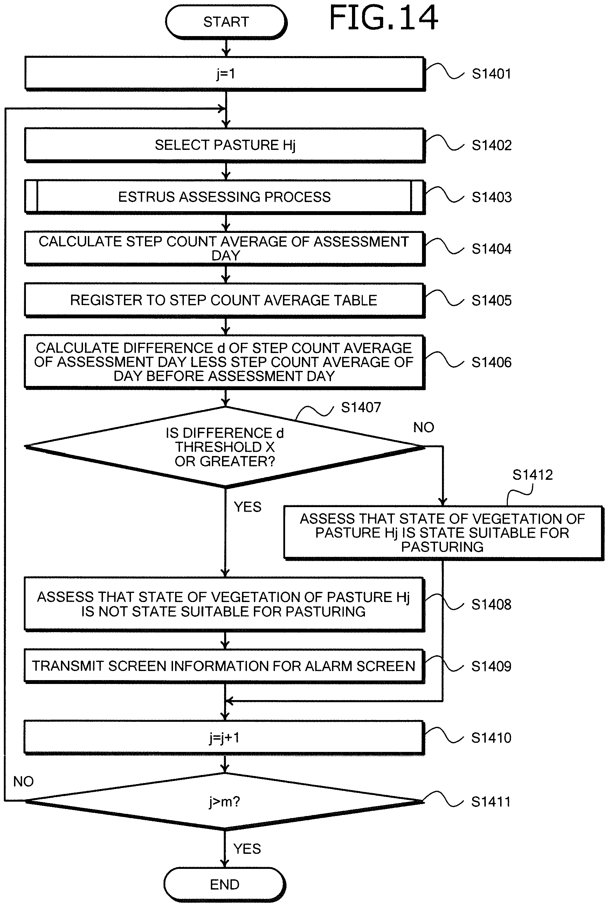

FIG. 14 is a flowchart depicting an example of a procedure of the vegetation assessing process of the vegetation assessing apparatus 101. In the flowchart depicted in FIG. 14, the vegetation assessing apparatus 101 sets "j" of the pasture Hj to "j=1" (step S1401), and selects the pasture Hj from among the pastures H1 to Hm (step S1402).

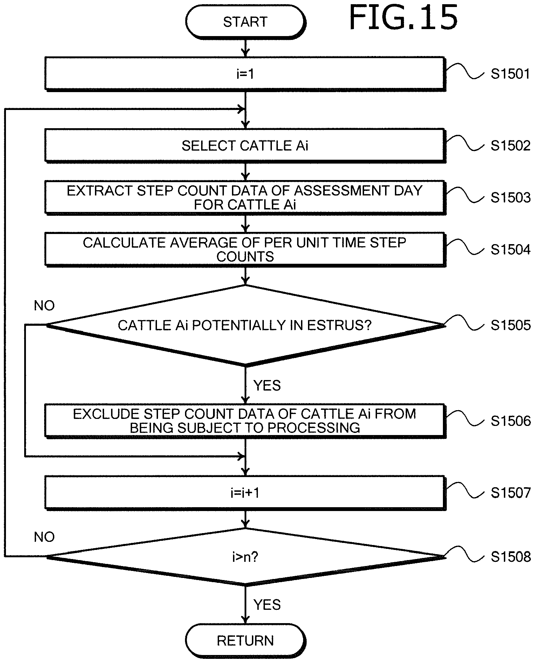

The vegetation assessing apparatus 101 executes an estrus assessing process (step S1403). The estrus assessing process is a process of assessing the possibility of a cattle Ai pastured in the pasture Hj being in estrus. A procedure of the estrus assessing process will be described hereinafter with reference to FIG. 15.

The vegetation assessing apparatus 101 calculates the step count average of the assessment day, based on the total step counts of the assessment day, for the remaining cattle among the cattle A1 to An pastured in the pasture Hj exclusive of cattle potentially in estrus (step S1404). The vegetation assessing apparatus 101 registers the calculated step count average of the assessment day into the step count average table 1100 (step S1405).

The vegetation assessing apparatus 101 refers to the step count average table 1100 and calculates the difference d of the step count average of the assessment day less the step count average of the day before the assessment day (step S1406). The vegetation assessing apparatus 101 determines if the calculated difference d is the threshold X or greater (step S1407).

Here, if the difference d is the threshold X or greater (step S1407: YES), the vegetation assessing apparatus 101 assesses that the state of the vegetation of the pasture Hj is not a state suitable for pasturing (step S1408). The vegetation assessing apparatus 101 generates screen information for an alarm screen and transmits the screen information to the client apparatus 201 (step S1409). The alarm screen is a screen indicating that the state of the vegetation of the pasture Hj is not a state suitable for pasturing and, for example, is the alarm screen 1200 depicted in FIG. 12.

The vegetation assessing apparatus 101 increments "j" of the pasture Hj (step S1410), and determines whether "j" is greater than "m" (step S1411). Here, if "j" is "m" or less (step S1411: NO), the vegetation assessing apparatus 101 returns to step S1402.

On the other hand, if "j" is greater than "m" (step S1411: YES), the vegetation assessing apparatus 101 ends the series of operations according to the flowchart. At step S1407, if the difference d is less than the threshold X (step S1407: NO), the vegetation assessing apparatus 101 assesses that the state of the vegetation of the pasture Hj is a state suitable for pasturing (step S1412), and transitions to step S1410.

Thus, the state of the vegetation can be assessed for each pasture Hj, based on step count measurement results of a cattle Ai, for each of the pastures Hj located at multiple locations.

### <Procedure of estrus assessing process>

A procedure of the estrus assessing process at step S1403 depicted in FIG. 14 will be described.

FIG. 15 is a flowchart depicting an example of a procedure of the estrus assessing process. In the flowchart depicted in FIG. 15, the vegetation assessing apparatus 101 sets "i" of a cattle Ai pastured in a pasture Hj to "i=1" (step S1501), and selects the cattle Ai from among the cattle A1 to An pastured in the pasture Hj (step S1502).

The vegetation assessing apparatus 101 extracts from the step count table 230, step count data of the assessment day, for the cattle Ai (step S1503). The vegetation assessing apparatus 101 calculates an average of the per unit time step counts, based on the extracted step count data of the assessment day, for the cattle Ai (step S1504).

The vegetation assessing apparatus 101 determines if the calculated average of the step counts is the threshold α or greater and thereby, determines whether the cattle Ai has the potential of being in estrus (step S1505). Here, if the cattle Ai has no potential of being in estrus (step S1505: NO), the vegetation assessing apparatus 101 transitions to step S1507.

On the other hand, if the cattle Ai has the potential of being in estrus (step S1505: YES), the vegetation assessing apparatus 101 excludes the step count data of the cattle Ai from being subject to processing (step S1506). More specifically, for example, the vegetation assessing apparatus 101 may delete from the step count table 230, the step count data of the cattle Ai that has a potential of being in estrus.

The vegetation assessing apparatus 101 increments "i" of the cattle Ai (step S1507), and determines whether "i" is greater than "n" (step S1508). Here, if "i" is "n" or less (step S1508: NO), the vegetation assessing apparatus 101 returns to step S1502.

On the other hand, if "i" is greater than "n" (step S1508: YES), the vegetation assessing apparatus 101 ends the series of operations according to the flowchart and returns to the step at which the estrus assessing process was called.

Thus, the step count data of a cattle Ai that has a potential of being in estrus among the cattle A1 to An pastured in a pasture Hj can be excluded from being subject to processing when assessing whether the state of the vegetation of the pasture Hj is a state suitable for pasturing.

As described, the vegetation assessing apparatus 101 according to the embodiment enables assessment of whether the state of vegetation of a pasture Hj is a state suitable for pasturing, based on temporal changes of step count measurement results obtained by the communications apparatus 203 attached to a cattle Ai pastured in the pasture Hj. Further, the vegetation assessing apparatus 101 enables obtained assessment results to be transmitted to the client apparatus 201 used by the livestock raiser.

Thus, without having to go to the pasture Hj to observe the vegetation, the livestock raiser can determine the state of the vegetation of the pasture Hj, enabling the work load of the livestock raiser to be reduced. Further, even if the livestock raiser does not have knowledge related to the vegetation of a meadow, the livestock raiser can determine the state of the vegetation of the pasture Hj, enabling personnel costs for management of the pasture Hj to be greatly suppressed.

The vegetation assessing apparatus 101 further enables assessment that the state of the vegetation of a pasture Hj is not a state suitable for pasturing, when the step count measurement results for each given time interval T increase with the elapse of time. Thus, when the step count increases consequent to little feed grass in a pasture Hj or the cattle Ai searching for preferred grass since the feed grass has been depleted, the state of the vegetation of the pasture Hj can be assessed to not be a state suitable for pasturing.

The vegetation assessing apparatus 101 enables classification of step count measurement results according to each pasture Hj identified from information related to the location of a cattle Ai. The vegetation assessing apparatus 101 further enables assessment of whether the state of the vegetation of each pasture Hj is a state suitable for pasturing, based on temporal changes of the step count measurement results for each pasture Hj. Thus, the state of vegetation of the respective pastures Hj located at multiple locations can be assessed.

The vegetation assessing apparatus 101 further enables assessment of whether the state of the vegetation of a pasture Hj is suitable for pasturing, based on temporal changes of the step count measurement results of the remaining cattle among the cattle A1 to An pastured in the pasture Hj exclusive of cattle of a specified state. As a result, the step count data of a cattle whose per unit time step count, compared to a normal state, is increasing consequent to factors other than preferences for feed grass, such as estrus and illness can be excluded from being subject to processing, thereby enabling drops in the accuracy to be prevented when assessment of the state of the vegetation of a pasture Hj is performed.

The vegetation assessing apparatus 101 enables assessment that the state of the vegetation of a pasture Hj is not a state suitable for pasturing, when the difference d of the step count average of the assessment day less the step count average of the day before the assessment day is the threshold X or greater. As a result, the state of the vegetation of a pasture Hj can be assessed to not be a state suitable for pasturing, when the step count of a cattle Ai pastured in the pasture Hj rapidly increases.

The vegetation assessing apparatus 101 enables the time slot TZ, for which the step count continues to be the given number Q or less for the given period P or longer, to be detected based on the step count measurement results of a cattle Ai, for each time slot of the assessment day. Further, the vegetation assessing apparatus 101 enables assessment that the state of the vegetation of a pasture Hj is not a state suitable for pasturing, when the count k of the detected time slots TZ is less than the given number K.

As a result, compared to a case where only the extent of increase of the step counts of each given time interval T is considered, the accuracy in assessing the state of the vegetation of a pasture Hj can be improved. For example, when the step count of a cattle Ai rapidly increases consequent to an external factor such as thunder, the appearance of a natural predator, etc., an assessment that the state of the vegetation of the pasture Hj is not a state suitable for pasturing can be prevented.

Thus, the vegetation assessing program, the vegetation assessing apparatus, and the vegetation assessing method according to the embodiment enable a livestock raiser to comprehend the state of the vegetation of a pasture Hj and take measures according to the state of the vegetation of the pasture Hj. More specifically, for example, when the state of the vegetation of the pasture Hj has become poor, the livestock raiser can quickly take countermeasures such as sowing in the pasture Hj, seeds of grasses highly preferred by the cattle Ai, changing the pasture to be used for pasturing, etc., thereby enabling productivity to be increased.

The vegetation assessing method described in the present embodiment may be implemented by executing a prepared program on a computer such as a personal computer and a workstation. The vegetation assessing program is stored on a, computer-readable recording medium such as a hard disk, a flexible disk, a CD-ROM, an MO, and a DVD, read out from the computer-readable medium, and executed by the computer. The program may be distributed through a network such as the Internet.

### EXPLANATIONS OF LETTERS OR NUMERALS

- 101: vegetation assessing apparatus
- 102: step measuring device
- 103: communications terminal
- 200: rearing support system
- 201: client apparatus
- 202: relay apparatus
- 203: communications apparatus
- 1001: obtaining unit
- 1002: assessing unit
- 1003: output unit

## Claims

1. A vegetation assessing program that causes a computer to execute a process comprising:
assessing whether a state of vegetation of a pasture is a state suitable for pasturing, based on temporal changes of step count measurement results obtained by a step measuring device attached to a livestock animal pastured in the pasture; and
outputting an obtained assessment result.

2. The vegetation assessing program according to claim 1, wherein
the assessing includes assessing that the state of the vegetation of the pasture is not a state suitable for pasturing, when the step count measurements for each given time interval increase with an elapse of time.

3. The vegetation assessing program according to claim 1 or 2, the process further comprising
classifying the step count measurement results according to pasture, based on information related to a location of the livestock animal, wherein
the assessing includes assessing whether the state of the vegetation of the pasture is a state suitable for pasturing, based on the step count measurement results classified according to pasture.

4. The vegetation assessing program according to any one of claims 1 to 3, wherein
the assessing includes assessing whether the state of the vegetation of the pasture is a state suitable for pasturing, based on temporal change of the step count measurement results of a remaining livestock animal among a plurality of livestock animals for which a step count has been measured exclusive of a livestock animal of a specified state.

5. The vegetation assessing program according to any one of claims 1 to 4, wherein
the assessing includes assessing that the state of the vegetation of the pasture is not a state suitable for pasturing, when a value of the step count measurement results of a first interval less the step count measurement results of a second interval that is before the first interval is a threshold or greater.

6. The vegetation assessing program according to claim 5, the process further comprising
detecting based on the step count measurement results for each time slot in the first interval, a time slot for which the step count continues to be a given number or less for a given period or longer, when the value is the threshold or greater, wherein
the assessing includes assessing that the state of the vegetation of the pasture is not a suitable state for pasturing, when a count of the detected time slot is less than a given count.

7. A vegetation assessing apparatus comprising:
an assessing unit (1002) configured to assess whether a state of vegetation of a pasture is a state suitable for pasturing, based on temporal change of step count measurement results obtained by a step measuring device attached to a livestock animal pastured in the pasture; and
an output unit (1003) configured to output an assessment result obtained by the assessing unit (1002).

8. A vegetation assessing method comprising:
assessing whether a state of vegetation of a pasture is a state suitable for pasturing, based on temporal change of step count measurement results obtained by a step measuring device attached to a livestock animal pastured in the pasture; and
outputting an obtained assessment result, wherein
the vegetation assessing method is executed by a computer.

## Patentansprüche

1. Vegetationsbeurteilungsprogramm, das einen Computer veranlasst, einen Prozess auszuführen, der Folgendes umfasst:
Beurteilen, ob ein Vegetationszustand einer Weide ein Zustand ist, der zum Weiden geeignet ist, basierend auf zeitlichen Änderungen von Schrittzählungsmessungsresultaten, die von einer Schrittzählungsvorrichtung, die an einem Nutztier, das auf der Weide weidet, befestigt ist, erzielt werden, und
Ausgeben eines erhaltenen Beurteilungsresultats.

2. Vegetationsbeurteilungsprogramm nach Anspruch 1, wobei
die Beurteilung das Beurteilen aufweist, dass der Zustand der Vegetation der Weide kein Zustand ist, der zum Weiden geeignet ist, wenn die Schrittzählungsmessungen für jedes gegebene Zeitintervall um eine Zeitspanne zunehmen.

3. Vegetationsbeurteilungsprogramm nach Anspruch 1 oder 2, wobei der Prozess Folgendes umfasst:
Einstufen der Schrittzählungsmessungsresultate gemäß der Weide, basierend auf Informationen in Zusammenhang mit einem Ort des Nutztiers, wobei
die Beurteilung das Beurteilen aufweist, ob der der Zustand der Vegetation der Weide ein Zustand ist, der zum Weiden geeignet ist, basierend auf den Schrittzählungsmessungsresultaten, die gemäß der Weide eingestuft sind.

4. Vegetationsbeurteilungsprogramm nach einem der Ansprüche 1 bis 3, wobei
die Beurteilung das Beurteilen aufweist, ob der Zustand der Vegetation der Weide ein Zustand ist, der zum Weiden geeignet ist, basierend auf zeitlicher Änderung der Schrittzählungsmessungsresultate eines restlichen Nutztiers einer Mehrzahl von Nutztieren, für das eine Schrittzählung ausschließlich eines Nutztiers mit einem spezifizierten Zustand gemessen wurde.

5. Vegetationsbeurteilungsprogramm nach einem der Ansprüche 1 bis 4, wobei
die Beurteilung das Beurteilen aufweist, dass der Zustand der Vegetation der Weide kein Zustand ist, der zum Weiden geeignet ist, wenn ein Wert der Schrittzählungsmessungsresultate eines ersten Intervalls abzüglich der Schrittzählungsmessungsresultate eines zweiten Intervalls, das vor dem ersten Intervall liegt, ein Schwellenwert oder größer ist.

6. Vegetationsbeurteilungsprogramm nach Anspruch 5, wobei der Prozess ferner Folgendes umfasst:
Erfassen, basierend auf den Schrittzählungsmessungsresultaten für jeden Zeitschlitz in dem ersten Intervall, eines Zeitschlitzes, für den der Schrittzählung weiterhin eine Anzahl oder weniger für eine gegebene Periode oder länger gegeben wird, wenn der Wert der Schwellenwert oder größer ist, wobei
die Beurteilung das Beurteilen aufweist, dass der Zustand der Vegetation der Weide kein geeigneter Zustand zum Weiden ist, wenn eine Zählung des erfassten Zeitschlitzes geringer ist als eine gegebene Zählung.

7. Vegetationsbeurteilungsgerät, das Folgendes umfasst:
eine Beurteilungseinheit (1002), die konfiguriert ist, um zu beurteilen, ob ein Vegetationszustand einer Weide ein Zustand ist, der zum Weiden geeignet ist, basierend auf einer zeitlichen Änderung von Schrittzählungsmessungsresultaten, die durch eine Schrittzählungsvorrichtung, die an einem Nutztier, das auf der Weide weidet, befestigt ist, erhalten werden, und
eine Ausgabeeinheit (1003), die konfiguriert ist, um ein Beurteilungsresultat auszugeben, das von der Beurteilungseinheit (1002) erhalten wird.

8. Vegetationsbeurteilungsverfahren, das Folgendes umfasst:
Beurteilen, ob ein Vegetationszustand einer Weide ein Zustand ist, der zum Weiden geeignet ist, basierend auf zeitlicher Änderung von Schrittzählungsmessungsresultaten, die durch eine Schrittmessungsvorrichtung, die an einem Nutztier, das auf der Weide weidet, befestigt ist, erhalten werden, und
Ausgeben eines erhaltenen Beurteilungsresultats, wobei
das Vegetationsbeurteilungsverfahren von einem Computer ausgeführt wird.

## Revendications

1. Programme d'évaluation de végétation qui amène un ordinateur à exécuter un processus comprenant :
l'évaluation du fait qu'un état de végétation d'un pâturage est un état approprié pour le pâturage, sur la base de changements temporels de résultats de mesure de nombre de pas obtenus par un dispositif de mesure de pas attaché à un animal d'élevage en pâture dans le pâturage ; et
la fourniture en sortie d'un résultat d'évaluation obtenu.

2. Programme d'évaluation de la végétation selon la revendication 1, dans lequel l'évaluation comporte l'évaluation du fait que l'état de la végétation du pâturage n'est pas un état approprié pour le pâturage, quand les mesures de nombre de pas pour chaque intervalle de temps donné augmentent avec un écoulement du temps.

3. Programme d'évaluation de végétation selon la revendication 1 ou 2, le processus comprenant en outre
la classification des résultats de mesure de nombre de pas selon le pâturage, sur la base d'informations liées à un emplacement de l'animal d'élevage, dans lequel l'évaluation comporte l'évaluation du fait que l'état de la végétation du pâturage est un état approprié pour le pâturage, sur la base des résultats de mesure de nombre de pas classés selon le pâturage.

4. Programme d'évaluation de végétation selon l'une quelconque des revendications 1 à 3, dans lequel
l'évaluation comporte l'évaluation du fait que l'état de la végétation du pâturage est un état approprié pour le pâturage, sur la base d'un changement temporel des résultats de mesure de nombre de pas d'un animal d'élevage restant parmi une pluralité d'animaux d'élevage pour lesquels un nombre de pas a été mesuré en excluant un animal d'élevage d'un état spécifié.

5. Programme d'évaluation de végétation selon l'une quelconque des revendications 1 à 4, dans lequel
l'évaluation comporte l'évaluation du fait que l'état de la végétation du pâturage n'est pas un état approprié pour le pâturage, quand une valeur des résultats de mesures de nombre de pas d'un premier intervalle moins les résultats de mesure de nombre de pas d'un deuxième intervalle qui est avant le premier intervalle est un seuil ou plus grande.

6. Programme d'évaluation de végétation selon la revendication 5, le processus comprenant en outre
la détection, sur la base des résultats de mesures de nombre de pas pour chaque tranche temporelle dans le premier intervalle, d'une tranche temporelle pour laquelle le nombre de pas continue à être un nombre donné ou moins pour une période donnée ou plus longue, quand la valeur est le seuil ou plus grande, dans lequel
l'évaluation comporte l'évaluation du fait que l'état de la végétation du pâturage n'est pas un état approprié pour le pâturage, quand un nombre de la tranche temporelle détectée est inférieur à un nombre donné.

7. Appareil d'évaluation de végétation comprenant :
une unité d'évaluation (1002) configurée pour évaluer le fait qu'un état de végétation d'un pâturage est un état approprié pour le pâturage, sur la base d'un changement temporel de résultats de mesure de nombre de pas obtenus par un dispositif de mesure de pas attaché à un animal d'élevage en pâture dans le pâturage ; et
une unité de sortie (1003) configurée pour fournir en sortie un résultat d'évaluation obtenu par l'unité d'évaluation (1002).

8. Procédé d'évaluation de végétation comprenant :
l'évaluation du fait qu'un état de végétation d'un pâturage est un état approprié pour le pâturage, sur la base d'un changement temporel de résultats de mesure de nombre de pas obtenus par un dispositif de mesure de pas attaché à un animal d'élevage en pâture dans le pâturage ; et
la fourniture en sortie d'un résultat d'évaluation obtenu, dans lequel le procédé d'évaluation de végétation est exécuté par un ordinateur.
